(19) Europäisches Patentamt / European Patent Office / Office européen des brevets

(11) **EP 3 033 640 B1**

(12) **EUROPÄISCHE PATENTSCHRIFT**

(45) Veröffentlichungstag und Bekanntmachung des Hinweises auf die Patenterteilung:
**05.06.2019 Patentblatt 2019/23**

(21) Anmeldenummer: **14749768.9**

(22) Anmeldetag: **04.08.2014**

(51) Int Cl.:
**G02B 3/02** (2006.01)   **A61F 2/16** (2006.01)

(86) Internationale Anmeldenummer:
**PCT/EP2014/066700**

(87) Internationale Veröffentlichungsnummer:
**WO 2015/022217 (19.02.2015 Gazette 2015/07)**

(54) **AUGENLINSE MIT EINEM SPEZIFISCH GEFORMTEN ÜBERGANGSBEREICH EINES OPTISCHEN TEILS**

OCULAR LENS HAVING A SPECIFICALLY SHAPED OPTICAL TRANSITION REGION

LENTILLE OCULAIRE POURVUE D'UNE ZONE DE TRANSITION DE FORME SPÉCIFIQUE D'UN ÉLÉMENT OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.08.2013 DE 102013216014**

(43) Veröffentlichungstag der Anmeldung:
**22.06.2016 Patentblatt 2016/25**

(73) Patentinhaber: **Carl Zeiss Meditec AG**
**07745 Jena (DE)**

(72) Erfinder:
• **GERLACH, Mario**
**16548 Glienicke-Nordbahn (DE)**
• **BÖHME, Beate**
**07751 Großpürschütz (DE)**
• **DOBSCHAL, Hans-Jürgen**
**99510 Kleinromstedt (DE)**

(74) Vertreter: **Hofstetter, Schurack & Partner**
**Patent- und Rechtsanwaltskanzlei**
**PartG mbB**
**Balanstrasse 57**
**81541 München (DE)**

(56) Entgegenhaltungen:
**WO-A1-03/009053     WO-A1-2013/045079**

**Beschreibung**

Die Erfindung betrifft eine Augenlinse, insbesondere eine Intraokularlinse, mit einem optischen Teil, der eine erste optische Oberfläche aufweist.

Stand der Technik

Augenlinsen in Form von Intraokularlinsen weisen einen optischen Teil auf, an dem üblicherweise ein haptischer Teil anschließt. Mit dem haptischen Teil wird die Intraokularlinse im Kapselsack des Auges gehalten. Der optische Teil kann in vielfältiger Weise gestaltet werden, um die entsprechenden Sehfehler eines menschlichen Auges korrigieren zu können. In dem Zusammenhang können die Oberflächen des optischen Teils sphärisch oder asphärisch ausgebildet sein. Es sind auch torische Augenlinsen bekannt.

Gerade bei sehr komplexen Oberflächenformen des optischen Teils ist die Fertigung der Augenlinse schwierig und aufwändig. Das Fertigungswerkzeug muss äußerst präzise an dem Linsenmaterial ansetzen, um keine unerwünschten Formen zu generieren, welche dann die optischen Eigenschaften der Linse beeinträchtigen würden.

Da jedoch dem Fertigungsprozess mit einem derartigen Werkzeug im Hinblick auf die theoretisch bestimmte und berechnete Augenlinse im Hinblick auf die Erzeugung eines expliziten derartigen Optimums Grenzen gesetzt sind, ist dem Rechnung zu tragen. So treten in der Praxis häufig Probleme bei sehr gezackten oder diskreten Stufen auf der Oberfläche des optischen Teils bei der Fertigung auf. Denn hier entstehen sehr große Beschleunigungen am Fertigungswerkzeug, die zu entsprechenden Problemen bei der Materialbearbeitung der Augenlinse führen können.

Aus der WO 2013/045079 A1 ist eine Intraokularlinse bekannt, welche auf einem optischen Teil eine spiralförmige Oberflächenstruktur aufweist.

Darstellung der Erfindung

Es ist Aufgabe der vorliegenden Erfindung, eine Augenlinse im optischen Teil derart zu gestalten, dass zumindest eine gleichbleibende optische Abbildungseigenschaft bei verbesserter Fertigbarkeit erzielt werden kann.

Diese Aufgabe wird durch eine Augenlinse gemäß dem unabhängigen Anspruch gelöst.

Eine erfindungsgemäße Augenlinse umfasst einen optischen Teil, der zumindest eine erste optische Oberfläche aufweist. Diese erste optische Oberfläche ist in radialer Richtung betrachtet zumindest bereichsweise als Windung mit einer Ganghöhe umlaufend um eine Hauptachse der Augenlinse ausgebildet. Die Hauptachse ist die optische Hauptachse der Augenlinse. Diese erste optische Oberfläche ist somit zumindest bereichsweise als Schraubenbahn um die Hauptachse umlaufend ausgebildet. Durch eine derartige Windung der Oberfläche und der erzeugten Ganghöhe tritt somit in Richtung der Hauptachse ein gewisser Versatz zwischen einem Anfang und einem Ende der Windung auf. Zwischen diesem Anfang und dem Ende der Windung ist ein Übergangsbereich der optischen Oberfläche ausgebildet. Dieser Übergangsbereich ist mit einem Anfangsrand und einem Endrand in die Windung übergehend gestaltet. Der Anfangsrand erstreckt sich zwischen der Hauptachse und einer ersten Umfangsstelle des optischen Teils und der Endrand erstreckt sich zwischen der Hauptachse und einer zweiten Umfangsstelle. Der in eine Ebene senkrecht zur Hauptachse projizierte Anfangsrand weist einen nicht-geradlinigen Verlauf auf, wobei zusätzlich oder anstatt dazu der in eine Ebene senkrecht zur Hauptachse projizierte Anfangsrand des Übergangsbereichs einen nicht-geradlinigen Verlauf aufweist. Eine derart spezifisch gewundene optische Oberfläche weist zunächst zwischen ihrem Anfang und ihrem Ende diesen Übergangsbereich auf. Diesen im Hinblick auf bestmögliche optische Abbildungseigenschaften der ersten optischen Oberfläche und bestmögliche Fertigbarkeit zu optimieren, ist ein wesentlicher Fokus der Erfindung. In dem Zusammenhang ist ein abrupter vollständig gestufter Übergang mit einer quasi vertikalen Wand zwischen dem Anfang und dem Ende im Hinblick auf eine theoretische Darstellung der Augenlinse der optimalste Übergang. Da ein derartiger in der Fertigung nicht ausgebildet werden kann, ist es ein wesentlicher Aspekt der Erfindung, diesen Übergangsbereich formspezifisch so zu gestalten, dass im Hinblick auf die optische Abbildungseigenschaft der Augenlinse und gleichzeitig präzise Fertigbarkeit ein Maximum erzielt ist.

Dies wird durch die ganz gezielte eckenfreie Gestaltung des Übergangsbereichs mit einer eckenfreien Einmündung in den Anfang und das Ende der Windung erzeugt. In besonders hervorzuhebender Weise ist diese Aufgabenlösung jedoch durch den ganz spezifischen Verlauf des Anfangsrands und des Endrands des Übergangsbereichs erzielt. Durch eine derartige spezifische Ausgestaltung kann der Übergangsbereich praktisch minimal dimensioniert werden und die gewünschten optischen Eigenschaften der optischen Oberfläche im Vergleich zum theoretischen Optimum quasi maximiert werden und darüber hinaus auch die Fertigungsexaktheit der simulierten Linse wesentlich verbessert werden. Da durch diese detaillierte spezifizierte Dimensionierung und Formgestaltung des Übergangsbereichs das Fertigungswerkzeug keinen unerwünschten abrupten hohen Beschleunigungen ausgesetzt wird, kann die Übergangsbereichsgestaltung durch das Werkzeug äußerst präzise und genau erfolgen. Indem nunmehr keine unerwünschten und abrupten Beschleunigungen mehr für das Werkzeug auftreten, können auch unerwünschte Materialabtragungen oder nicht ausreichend abgetragenes Material bei der Linsenfertigung vermieden werden.

Es kann vorgesehen sein, dass die gesamte optische Oberfläche als gewundene Struktur ausgebildet ist, die eine vollständige Windung oder mehrere Windungen um die Hauptachse aufweist. Bei dieser Ausführung erstreckt sich dann der Anfangsrand direkt beginnend von der Hauptachse bis zur Umfangsstelle und somit über die gesamte Länge zwischen diesen Endpunkten. Entsprechendes gilt dann für den Endrand.

Es kann jedoch auch vorgesehen sein, dass die optische Oberfläche neben einem ersten Bereich, der eine Windung oder mehrere Windungen aufweist, einen zweiten Bereich umfasst, der keine gewundene Struktur aufweist. Beispielsweise kann hier vorgesehen sein, dass der zweite Bereich ein mittiger zentraler Bereich ist, der eine sphärische oder asphärische Oberfläche aufweist, an den sich radial der erste Bereich anschließt. Der erste Bereich kann dann beispielsweise eine monofokale Linse sein. Bei dieser Ausführung erstreckt sich dann der Anfangsrand beginnend an der Umfangsstelle zwischen der Umfangsstelle und bis zu dem Punkt, an dem in radialer Richtung betrachtet der erste Bereich endet. Der Anfangsrand ist zwar dann auch zwischen der Hauptachse und der Umfangsstelle erstreckend ausgebildet, erstreckt sich jedoch nicht ganz bis zur Hauptachse hin und endet somit beabstandet zur Hauptachse. Selbiges gilt dann für den Endrand.

Die Ganghöhe ist die Höhe der Windung in Richtung der Hauptachse zwischen dem Anfang und dem Ende.

Vorzugsweise ist vorgesehen, dass der Anfangsrand und/oder der Endrand über die gesamte Länge gekrümmt ist bzw. gekrümmt sind. Eine derartige Ausgestaltung zumindest eines Rands begünstigt die oben genannten Vorteile nochmals. Es ist somit ein sehr kontinuierlicher und gleichmäßiger Formverlauf eines Rands gegeben, wodurch keine abrupten Stufen und somit auch keine abrupten Beschleunigungs- und somit Richtungswechsel des Fertigungswerkzeugs gegeben sind. Gerade die Verbindung eines nicht-geradlinigen Anfangsrands und/oder Endrands in Projektion auf die Hauptebene mit einem eckenfreien und stetig in eine Richtung gekrümmten Verlauf eines derartigen Rands begünstigt die oben genannten Vorteile wesentlich.

Vorzugsweise ist vorgesehen, dass der Anfangsrand einen nicht-geradlinigen Verlauf aufweist, der gegenüber einer geradlinigen Verbindung zwischen der Hauptachse und der ersten Umfangsstelle in Umlaufrichtung um die Hauptachse betrachtet in Richtung des Endrands hin verlaufend ausgebildet ist. Zusätzlich oder anstatt dazu kann vorgesehen sein, dass der Endrand einen nicht-geradlinigen Verlauf aufweist, der gegenüber einer geradlinigen Verbindung zwischen der Hauptachse und der zweiten Umfangsstelle in Umlaufrichtung um die Hauptachse betrachtet in Richtung des Anfangsrands hin verlaufend ausgebildet ist. Gerade diese Krümmungsrichtungen der Ränder ermöglichen, den Übergangsbereich so klein wie möglich zu gestalten und somit die optischen Abbildungseigenschaften der Linse an das theoretische Optimum hin zu verbessern. Gleichzeitig wird die Fertigungsgenauigkeit jedoch auch positiv begünstigt.

Vorzugsweise ist vorgesehen, dass eine Oberfläche des Übergangsbereichs so geformt ist, dass zumindest am Anfangsrand, insbesondere über seine gesamte Länge, zwischen der Hauptachse und der erste Umfangsstelle ein Verlauf ausgebildet ist, bei dem die in Umlaufrichtung betrachtete Steigung der Oberfläche an allen radialen Stellen des Anfangsrands den gleichen Steigungsverlauf aufweist. Die Kontur bzw. der Konturenverlauf der Oberfläche ist somit dahingehend gestaltet, dass an dem Anfangsrand der mathematische funktionale Steigungsverlauf quasi über die gesamte radiale Länge des Anfangsrands gleich ist. Somit ist an jeder radialen Stelle des Anfangsrands die diesbezügliche Steigung in azimutaler Richtung betrachtet mit dem gleichen funktionalen Steigungsverlauf und somit der gleichen Beschreibungsfunktion für die Steigung ausgebildet. An den Stellen ist somit überall der gleiche Anstieg ausgebildet, was insbesondere bedeutet, dass die Beschleunigung für das Fertigungswerkzeug an diesen Stellen konstant ist, da die Beschleunigung mathematisch die Ableitung des Anstiegs darstellt. Durch diese ganz spezifische Formung der Oberfläche ist die Minimierung des Übergangsbereichs und somit gleichzeitig bestmöglicher optischer fertigbarer Abbildungseigenschaft der optischen ersten Oberfläche erreicht. Es ist praktisch ein Maximum der Parameter eines kleinen Übergangsbereichs, einer bestmöglichen Abbildungseigenschaft und genauer Fertigbarkeit des simulierten Übergangsbereichs erreicht.

Vorzugsweise ist vorgesehen, dass eine Oberfläche des Übergangsbereichs so geformt ist, dass zumindest am Endrand, insbesondere über seine gesamte Länge, zwischen der Hauptachse und der zweiten Umfangsstelle ein Verlauf ausgebildet ist, bei dem die in Umlaufrichtung betrachtete Steigung der Oberfläche an allen radialen Stellen des Endrands den gleichen Steigungsverlauf aufweist. Die oben genannten Vorteile gelten hier analog.

Vorzugsweise ist vorgesehen, dass eine Oberfläche des Übergangsbereichs einen Konturenverlauf in Umlaufrichtung betrachtet aufweist, der zumindest bereichsweise nicht-geradlinig ausgebildet ist. Insbesondere ist diese Oberflächengestaltung an allen radialen Stellen zwischen der Hauptachse und dem Umfang des optischen Teils entsprechend ausgebildet. Wird somit ein Schnitt an einer radialen Stelle im optischen Teil durch den Übergangsbereich gezogen, so ist der Konturenverlauf der Oberfläche über seine gesamte Länge nicht-geradlinig ausgebildet. Zum einen kann dadurch ein besonders kontinuierlicher und stufenloser Übergang zu den Enden der Windung der optischen ersten Oberfläche ermöglicht werden, andererseits eine im Hinblick auf die optischen Eigenschaften und die Fertigungsmöglichkeiten verbesserte Geometrie des Übergangsbereichs erzielt werden.

Vorzugsweise ist vorgesehen, dass der Konturenverlauf zumindest bereichsweise als eine Parabel ausgebildet ist. Diese Formgebung des Konturenverlaufs ist besonders vorteilhaft, da es eine besonders minimal zu dimensionierende Ausgestaltung des Übergangsbereichs in azimutaler Ausdehnung ermöglicht. Eine derartige Formgebung ermöglicht somit einen sehr begrenzten Übergangsbereich mit minimalem Flächenanteil, wodurch die optischen Abbildungseigenschaften des optischen Teils im Hinblick auf das theoretische Optimum besonders angenähert werden können. Ferner ist die Fertigung eines derartigen Konturenverlaufs sehr exakt möglich.

Es kann auch vorgesehen sein, dass der Konturenverlauf zumindest bereichsweise durch eine kubische Funktion beschrieben ist oder zumindest bereichsweise einen Kreisbahnabschnitt darstellt. Diese beiden alternativen Formgebungen für den Konturenverlauf ermöglichen eine deutliche Verbesserung gegenüber den bekannten Ausgestaltungen im Hinblick auf die Verbesserung der optischen Abbildungseigenschaften und die gleichzeitige Verbesserung der Fertigbarkeit.

Es kann auch vorgesehen sein, dass der Konturenverlauf der Oberfläche des Übergangsbereichs zwischen dem Anfangsrand und dem Endrand symmetrisch zu einer in Umlaufrichtung um die Hauptachse jeweils im gleichen Winkelabstand zu dem Anfangsrand und dem Endrand gelegenen Symmetriefläche, insbesondere Symmetrieebene, die die Hauptachse und den Symmetriepunkt aufweist. Vorzugsweise bildet diese Symmetrieebene somit die Ausgangslage, von der aus dann die azimutale funktionale Formbeschreibung der optischen Oberfläche und somit die Beschreibung in Umlaufrichtung gegeben ist. Diese Symmetrieebene ist somit senkrecht zur Hauptebene der Augenlinse und somit zur Hauptebene des optischen Teils, auf der dann die Hauptachse senkrecht steht, orientiert.

Durch eine derartige symmetrische Ausgestaltung des Konturenverlaufs, der in radialer Richtung von der Hauptachse bis zur Umfangsstelle bzw. zum Umfang an allen Stellen entsprechend symmetrisch ausgebildet ist, können die Vorteile bezogen auf verbessertes optisches Abbildungsverhalten und verbesserte Fertigbarkeit begünstigt werden.

Vorzugsweise ist vorgesehen, dass der funktionale Steigungsverlauf und somit die mathematische Formelbeschreibung der Steigung zwischen dem Anfangsrand und der Symmetrieebene an allen radialen Stellen gleich ist. Dies betrifft denjenigen Bereich der optischen Oberfläche der mit zumindest einer Windung ausgebildet ist.

Insbesondere ist vorgesehen, dass ein in die senkrecht zur Hauptachse stehende Hauptebene projizierter und durch den projizierten Anfangsrand und den projizierten Endrand begrenzter Flächenbereich des Übergangsbereichs kleiner ist, als eine Fläche in dieser Hauptebene, die durch zwei geradlinige Flächenränder begrenzt ist, von denen sich einer von der Hauptachse durch die erste Umfangsstelle und der weitere von der Hauptachse durch die zweite Umfangsstelle erstreckt.

Vorzugsweise ist vorgesehen, dass ein in Richtung der Hauptachse betrachtet der Hauptebene näherliegende Endrand einen nicht-geradlinigen Verlauf aufweist.

Insbesondere ist vorgesehen, dass der in die Hauptebene projizierte Endrand zum projizierten Anfangsrand hin gekrümmt ist. Insbesondere ist dabei vorgesehen, dass ausgehend von der zweiten Umfangsstelle der Endrand zum projizierten Anfangsrand hin stetig gekrümmt ist. Vorzugsweise gilt das gleiche analog für den projizierten Anfangsrand in Bezug zum Endrand.

Die Oberfläche des Übergangsbereichs ist vollständig stufenlos ausgebildet.

Es ist insbesondere vorgesehen, dass die Oberfläche des Übergangsbereichs so gekrümmt und somit geformt ist, und der Endrand und der Anfangsrand so ausgebildet sind, dass die Steigung in allen Punkten des Anfangsrands über die Länge des Anfangsrands in radialer Richtung gesehen maximal um 10 % variiert, insbesondere gleich ist. Analoges gilt in vorteilhafter Weise für den Endrand.

Vorzugsweise ist vorgesehen, dass eine Asphärizität der ersten optischen Oberfläche um die Hauptachse umlaufend betrachtet variiert. Insbesondere ist hier vorgesehen, dass sie kontinuierlich variiert, insbesondere sich kontinuierlich vergrößert.

In vorteilhafter Weise ist vorgesehen, dass zumindest eine Oberfläche des optischen Teils so gestaltet ist, dass sich die Brechkraft des optischen Teils in Umlaufrichtung um die Hauptachse bei einer Umdrehung um einen Wert zwischen 1 Dioptrien und 5 Dioptrien, insbesondere zwischen 1 Dioptrien und 4 Dioptrien ändert, und/oder vorzugsweise kontinuierlich ändert. Bei einer einzigen vollständigen Windung und somit bei einem einzigen Umlauf wird somit insbesondere eine derartige Dioptrieänderung vollzogen.

Vorzugsweise wird dabei ausgehend von einem Anfangsdioptriewert, der zwischen 19 und 22 Dioptrien, vorzugsweise bei 20 Dioptrien liegt, ausgegangen. Bei einer einzigen Umdrehung wird dann eine oben genannte vorteilhafte Änderung der Dioptrie durchgeführt, so dass diese um das entsprechend angegebene vorteilhafte Intervall sich dann vergrößert.

Vorzugsweise ist vorgesehen, dass sich die Brechkraft des optischen Teils der Augenlinse außerhalb des Übergangsbereichs linear als Funktion vom Winkel in Umlaufrichtung um die Hauptachse ändert. Eine derartige Ausgestaltung ist besonders vorteilhaft im Hinblick auf die Ausbildung einer Tiefenschärfenlinse mit einem entsprechend erweiterten Fokusbereich, in dem dann die Brechkraftverteilung besonders gleichmäßig und harmonisch auftritt und somit das optische Abbildungsverhalten über einen entsprechenden Dioptriewert besonders begünstigt ist.

Vorzugsweise ist vorgesehen, dass der Übergangsbereich, insbesondere zwischen den beiden Umfangsstellen gemessen, in Umlaufrichtung eine Winkelbreite < 7°, insbesondere zwischen 5° und 6,5°, aufweist. Eine derartig schmale Dimensiorierung eines Übergangsbereichs ist durch die vorteilhafterweise oben genannten Formgebungen der Oberfläche sowie des Anfangsrands und des Endrands begünstigt.

Vorzugsweise ist vorgesehen, dass die erste optische Oberfläche des optischen Teils eine Form aufweist, die durch eine von einer Summe von Sinus-Funktionen abhängigen Funktion beschreibbar ist. Insbesondere ist somit der Ablauf der Windung und somit der Schraubenbahn nicht linear, sondern abhängig von Kosinus-Funktionen. Die oben genannten Vorteile bezüglich dem optischen Abbildungsverhalten, gerade im Hinblick auf die vorteilhafterweise genannten Dioptriewertänderungen, sind dadurch besonders geeignet.

Vorzugsweise ist vorgesehen, dass eine rückwärtige zweite optische Oberfläche des optischen Teils symmetrisch zur ersten optischen Oberfläche in Bezug zur senkrecht zur Hauptachse stehenden Hauptebene ausgebildet ist. Bei einer derartigen zweiseitigen Ausgestaltung mit einer jeweils einmal umlaufenden Windung mit einer entsprechenden Ganghöhe und einem entsprechenden Übergangsbereich ist es möglich, dass die Dioptriewertänderungen, wie sie beispielhaft oben bereits erwähnt wurden, auf beide Seiten und somit auf beide Oberflächen verteilt sind. Dies bedeutet, dass auf jeder Oberfläche nicht mehr die volle gewünschte Dioptriewertänderung bei einem einzigen vollständigen Umlauf erreicht werden muss, sondern diesbezüglich nur noch die Hälfte des Wertes der gewünschten Dioptriewertänderung erreicht werden muss. So kann auf beiden Seiten beispielsweise jeweils eine Dioptrieänderung von 10.5 auf 12 Dioptrie bei einer Windung vorgesehen sein, so dass in Summe eine Änderung von 21 auf 24 Dioptrie erfolgt.

Vorteilhafterweise ist die Oberflächengestaltung des optischen Teils derart, dass die Augenlinse eine refaktive Struktur aufweist.

Insbesondere ist die Windung nur einmal um die Achse umlaufend ausgebildet und erstreckt sich von der Hauptachse bis zum Umfang ohne mehrfache Wellen- und Tälerform.

Vorzugsweise ist vorgesehen, dass an dem optischen Teil zumindest eine Haptik anschließend angeordnet und ausgebildet ist, und eine Übergangszone eine Oberfläche aufweist, deren Kontur zumindest bereichsweise eine Kreisbogenform oder eine Parabelform aufweist. Insbesondere ist dabei der Radius dieses Kreisbogens azimutal veränderlich. Insbesondere ist vorgesehen, dass der Übergang zwischen der Kontur des optischen Teils und der Kontur des haptischen Teils und somit die Gestaltung der Übergangszone ohne einen geradlinigen Abschnitt ausgebildet ist.

Besonders vorteilhaft ist es, wenn die Augenlinse als Tiefenschärfelinse ausgebildet ist. Insbesondere ist sie eine Intraokularlinse. In besonders vorteilhafter Weise ist vorgesehen, dass die Winkelbereiche in Umlaufrichtung um die Hauptachse und somit die azimutalen Winkelbereiche im Hinblick auf eine Änderung der Brechkraft und somit der Dioptriewerte so aufgeteilt sind, dass in einem ersten Winkelbereich, insbesondere beginnend beim Anfang der Windung, zwischen 115° und 119°, insbesondere von 118° eine Dioptriewertänderung zwischen 0,5 und 1,5, insbesondere von 1, auftritt, in einem daran anschließenden weiteren Winkelbereich von nochmals zwischen 115° und 119°, insbesondere 118°, eine weitere Dioptriewertänderung zwischen 0,5 und 1,5, insbesondere von 1, auftritt, und insbesondere in einem dritten Winkelbereich von ebenfalls vorzugsweise zwischen 115° und 119°, insbesondere 118°, eine weitere Dioptriewertänderung zwischen 0,5 und 1,5, insbesondere von 1, auftritt, so dass in einem zusammenhängenden Winkelbereich von vorzugsweise 354° eine Dioptriewertänderung von 3 Dioptrien erreicht wird.

Es kann auch vorgesehen sein, dass eine Dioptriewertänderung größer 3 erfolgt, beispielsweise um einen Wert bis zu 3,7, so dass beispielsweise auch eine Änderung um 3,3, erfolgen kann.

Vorzugsweise ändert sich in diesem Winkelbereich der Windung außerhalb des Übergangsbereichs die Brechkraft linear als Funktion vom Azimutwinkel.

In vorteilhafter Weise ist vorgesehen, dass für eine vergrößerte Intensität in der Ferne und der Nähe der Augenlinse eine Dioptriewertänderung zwischen 0,3 und 0,7, insbesondere von 0,5, in einem ersten Winkelbereich, insbesondere beginnend am Anfang der Windung, zwischen 148° bis 154°, insbesondere von 152°, ausgebildet ist. In einem daran anschließenden zweiten Winkelbereich von vorzugsweise ebenfalls zwischen 148° bis 154°, insbesondere von 152° ist dann eine Dioptriewertänderung zwischen 1,8 und 2,2, insbesondere von weiteren 2 Dioptrien, vorgesehen, wobei hier bei dem Dioptriewert fortgefahren wird, der am Ende des ersten Winkelbereichs erreicht ist.

In einem an den zweiten Winkelbereich anschließenden dritten Winkelbereich, der sich vorzugsweise zwischen 46° und 60°, insbesondere auf 50°, erstreckt, wird dann eine weitere Dioptriewertänderung zwischen 0,3 und 0,7, insbesondere um 0,5 Dioptrien, ausgebildet, wobei auch hier vorzugsweise von dem Dioptriewert ausgegangen wird, der am Ende des zweiten Winkelbereichs erreicht ist.

Vorzugsweise ist auch hier der Übergangsbereich mit einer azimutalen Breite von 6° ausgebildet, die insbesondere am äußeren radialen Ende und somit zwischen den beiden Umfangsstellen bemessen ist.

Vorzugsweise ist vorgesehen, dass z-Werte und somit die Werte der Oberfläche der Augenlinse in der Richtung der Hauptachse als Funktion vom Winkel φ, der sich in Umlaufrichtung um die Hauptachse bemisst und den Azimutwinkel darstellt, allgemein als Summe von Sinus-Funktionen beschreibbar sind, wie dies nachfolgende Formel 1 zeigt:

$$z(r,\varphi) = z_{\max}(r) * z(\varphi) = z_{\max}(r) * \sum a_n \sin(n\varphi) \qquad (1)$$

Hierbei bezeichnet r den Radius, $\varphi$ den Azimutwinkel (mit $-\pi \leq \varphi \geq \pi$) und $a_n$ die Koeffizienten.

Im einfachsten Fall kann die Ganghöhe hier $z_{max}$ (r) = C * r$^2$ sein, jedoch aber auch asphärisch sein. C beschreibt hierbei eine Konstante und bestimmt die maximale Dioptriewertänderung eines vollständigen Umlaufs bzw. einer Windung, und die Funktion $z_w(r,\varphi)$ verläuft zwischen -1 und +1.

Wenn vorzugsweise der Winkel $\varphi$=0 in der Mitte des Übergangsbereichs und somit in der Symmetrieebene liegt, ergeben sich entsprechend vorteilhafte Berechnungen.

Vorzugsweise wird diese Windung bzw. diese Schraubenform der Oberfläche auf eine Basislinse, die sich durch die Funktion $z_0$ (r) beschreiben lässt, aufgebracht. Es ergibt sich dabei eine gesamte Beschreibung der Oberfläche, die sich durch nachfolgende Formel 2 beschreiben lässt:

$$z_{gesamt}(r,\varphi) = z_0(r) + z(r,\varphi) \qquad (2)$$

In besonders vorteilhafter Weise ist vorgesehen, dass zumindest eine optische Oberfläche des optischen Teils so gestaltet und geformt ist, dass auch ein Astigmatismus als Sehfehler des Auges korrigiert wird. Vorzugsweise ist dabei vorgesehen, dass die Oberfläche durch einen weiteren Summanden in der in Formel 2 angegebenen Gleichung beschrieben ist, wobei der Summand durch $a_2$*r$^2$*sin (2*$\varphi$) beschreibbar ist. Die optische Oberfläche ist zur Korrektur des Astigmatismus zumindest bereichsweise sattelartig bzw. torisch ausgebildet.

Vorzugsweise ist vorgesehen, dass die Sattelfläche, die sich durch den oben genannten Summanden für die Astigmatismuskorrektur ergibt, und die Oberfläche mit der Windung so zueinander ausgerichtet und zu einer Gesamtoberflächenform überlagert sind, dass ein oberer Sattelpunkt, welcher der höchste Punkt am Rand der Flächenform ist, azimutal in Umlaufrichtung mit der flachsten Krümmung - gleich dem Maximum im azimutalen Verlauf - der Windung in radialer Richtung zusammenfällt.

Insbesondere ist ein Rotationswinkel in Umlaufrichtung um die Achse zwischen der Sattelfläche und der Oberfläche mit der Windung so ausgebildet, dass das Maximum der die Sattelfläche beschreibenden Sinusfunktion des Summanden an der Stelle des flachsten Radius der Oberfläche mit der Windung und das Minimum der Sinusfunktion in Richtung des steilsten Radius der Oberfläche mit der Windung liegt bzw. der Nulldurchgang der Sinusfunktion in der Mitte des Übergangsbereich liegt, und die Sinus-Funktion fällt, wenn die Schrauben-Fläche im Übergangs-Bereich ebenfalls fällt.

Durch einen weiteren Summanden $b_2$*cos (2*$\varphi$) kann die Orientierung des Astigmatismus im Vergleich zum Übergang und somit zur Übergangszone beliebig geregelt werden. Vorteilhaft ist es, dass die Windung bzw. Schraubenform der Oberfläche so im Auge rotiert, dass der zweite Summand entfällt, und stattdessen der Koeffizient a2 entsprechend gewählt ist.

Wie bereits erwähnt, ist es vorteilhaft, den Konturenverlauf der Oberfläche des Übergangsbereichs zumindest bereichsweise durch eine Parabel als Grundform zu beschreiben. Dadurch kann erreicht werden, dass sich für die Geschwindigkeit und die Beschleunigung des Fertigungswerkzeugs und somit des Schneidmessers daraus ableitbare Relationen ergeben.

An die vorgegebene Parabel wird dann die gewünschte azimutale Windung und somit die Schraubenfunktion der optischen Oberfläche stetig angeschlossen, wobei hier im einfachsten Fall eine Gerade vorgesehen sein kann.

Durch diese Ausgestaltungen wird der Übergangsbereich mit den genannten Funktionsverläufen bei möglichst kleinen Absolutwerten der Beschleunigung geformt, so dass der Winkelbereich klein gehalten werden kann.

Es könnten jedoch auch ähnliche Kurvenverläufe vorgesehen sein, wobei beispielsweise zwischen den beiden Parabeln eines Konturenverlaufs des Übergangsbereichs wiederum eine lineare Funktion eingefügt ist. Weiter kann die Beschleunigung auch schrittweise auf die Maximalwerte steigen.

Alternativ ist eine lineare schraubenförmige Gestaltung der optischen Oberfläche mit einem kubischen Funktionsverlauf im Übergangsbereich möglich.

Um generell in besonders vorteilhafter Weise die Beschleunigung und somit die zweite Ableitung der die Oberfläche beschreibenden Funktion und andererseits den Übergangsbereich möglichst klein zu halten, ist die Funktion z (r,$\varphi$) derart von dem Radius abhängig zu gestalten, dass die Beschleunigung der Fertigungsmaschine zum Fertigen der Linse unabhängig vom Radius bleibt.

Insbesondere ist dies dadurch erreicht, in dem ein Umkehrpunkt der Parabeln, der durch den nachfolgenden Parameter d beschrieben wird, wie nachfolgend angegeben und in Abhängigkeit vom Radius festgelegt ist: Es wird hierbei e, welches den z-Wert des Umkehrpunkts bezeichnet, gleich der Funktion $z_{max}$ gesetzt:

$$e = e(r) = z_{max}(r) = C(r/r_0)^2. \qquad (3)$$

Dann wird eine feste Maximalbeschleunigung a für alle Radien vorgegeben. Im einfachsten Fall der oben angegeben radialen Funktion werden dann Umkehrpunkte erhalten, die in der Nähe der optischen Hauptachse einen kleineren Winkelabstand haben, der sich zu

$$d = d(r) = \sqrt{\frac{e(r)}{a}} = r^* \sqrt{\frac{C}{a * r_0^2}} \qquad (4)$$

ergibt.

Bei kleineren Radien und somit zur optischen Hauptachse hin vermindert sich dabei der WinkelAbstand der Umkehrpunkte.

Die Oberfläche des Übergangsbereichs hat damit die funktionale Beschreibung bzw. die Form:

$$z(r, \varphi) = z_{max(r)} * z(r,\varphi) = C\left(\frac{r}{r_0}\right)^2 * \left\{ C\left(\frac{r}{r_0}\right)^2 - a\left[\frac{r}{r_0} * \sqrt{\left\{\frac{C}{a}\right\}} - \varphi\right]^2 \right\} \qquad (5)$$

Die Funktion z $(r,\varphi)$ ist hier nicht mehr als Produkt einer Funktion vom Radius mit einer Funktion vom Winkel beschrieben, sondern die azimutale Funktion hängt insbesondere zusätzlich vom Winkel ab.

In vorteilhafter Ausführung ist vorgesehen, dass Krümmungsradius und Asphärizität auf der Vorder- und der Rückseite und somit der ersten optischen Oberfläche bzw. der zweiten optischen Oberfläche des optischen Teils der Augenlinse linear mit dem Azimutwinkel zwischen dem Wert einer Grundbrechkraft, die beispielsweise 21 Dioptrien betragen kann, und einer Summenbrechkraft variiert. Es kann insbesondere auch vorgesehen sein, dass nur die Vorder- oder die Rückseite variiert wird, wobei die Brechkraftdifferenz der Ausgangslinse dann doppelt so hoch gewählt wird. Entscheidend ist immer, dass der gewünschte Brechkraftänderungswert bei einem Umlauf der Schraubenbahn erzielt wird.

Insbesondere ist vorgesehen, dass die erste optische Oberfläche so gestaltet ist, dass nur eine einzige Windung einer Schraubenbahn ausgebildet ist, wobei sich somit auch die Schraubenbahn in radialer Richtung als refraktive Optik ausbildet.

Ein vorteilhafter Funktionsverlauf der z-Funktion ist dadurch gekennzeichnet, dass die zweite Ableitung einen Maximalwert nicht überschreitet und die Werkzeugmaschine zum Schneiden bzw. Fertigen der Augenlinse nur geringe Beschleunigungen in z-Richtung benötigt.

Bevorzugt wird dies durch den folgenden azimutalen Funktionsverlauf der optischen Oberfläche erreicht.

$$z(\varphi) = \left\{ \begin{array}{ll} m_1\varphi & 0 \le \varphi \le x_1, m_1 \rangle 0 \\ m_2\varphi - m_2 & x_2 \le \varphi \le 1, m_2 \langle 0 \\ -a * (\varphi - d)^2 + e & x_1 \le \varphi \le x_2, a \langle 0 \end{array} \right\} \qquad (6)$$

für $0 < \varphi < 1$: weiter gilt: $z(\varphi) = -z(-\varphi)$ für $-1 \le \varphi \le 0$

Bevorzugt sind hier die Parameter d und e wie in den Gleichungen 3 und 4 angegeben abhängig vom Radius.

Hier ist der Übergangsbereich durch zwei Parabeln mit umgekehrten Vorzeichen gekennzeichnet, die stetig aneinander anschließen, oder durch eine Gerade verbunden sind. Zu dieser Funktion kann der Summand $a_2$*sin $(2*\varphi)$ für den Astigmatismus addiert werden.

Die Funktion z $(\varphi)$ steigt beginnend bei einem Mittelwert $z(\varphi = 0) = 0$ linear mit dem Anstieg $m_1$ an, verläuft danach auf einer Parabel und fällt mit dem Anstieg $m_2$ wieder auf den Mittelwert.

In einer besonders bevorzugten Ausgestaltung kann der die Parabeln verbindende Geradenbereich mit dem Anstieg $m_1$ weggelassen werden, so dass der Übergang zwischen den beiden Parabeln bei x1 = 0 gleichwohl erfolgt, und sich ein besonders kleiner Übergangsbereich im Hinblick auf seine azimutale Ausdehnung ergibt. Für alle möglichen Funktionsverläufe, beispielsweise Parabelfunktion, kubische Funktion oder Kreisbahnabschnittfunktion können die jeweiligen Parameter der Variablen in Abhängigkeit vom Radius auf der Linse gewählt werden, so dass der Übergangsbereich insbesondere zur Hauptachse hin sehr klein wird.

Insbesondere ist auch vorgesehen, dass die Oberflächengestaltung der optischen Oberfläche auch für kleine Pupillen ($r < 1$) entsprechend angepasst und verkleinert werden kann.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind.

Kurze Beschreibung der Zeichnungen

Ausführungsbeispiele der Erfindung werden nachfolgend anhand schematischer Zeichnungen näher erläutert. Es zeigen:

Fig. 1a        eine perspektivische Darstellung eines ersten Ausführungsbeispiels einer erfindungsgemäßen Augenlinse;

Fig. 1b        eine perspektivische Darstellung eines weiteren Ausführungsbeispiels einer erfindungsgemäßen Augenlinse;

Fig. 2        eine perspektivische Darstellung eines Ausführungsbeispiels einer optischen Oberfläche eines optischen Teils eines Ausführungsbeispiels einer erfindungsgemäßen Augenlinse;

Fig. 3        einen vergrößerten Ausschnitt der Darstellung gemäß Fig. 2;

Fig. 4        eine andere Darstellung auf einen Übergangsbereich eines Ausführungsbeispiels einer erfindungsgemäßen Augenlinse;

Fig. 5        eine Projektionsdarstellung des Übergangsbereichs in eine Hauptebene der Augenlinse;

Fig. 6        ein Diagramm, bei dem der Oberflächenverlauf in Abhängigkeit des Azimutalwinkels für verschiedene Radien im Übergangsbereich dargestellt ist;

Fig. 7        ein Diagramm, bei dem die Ableitung der die Oberflächenform in Fig. 6 darstellenden mathematischen Funktion in Abhängigkeit vom Azimutwinkel dargestellt ist;

Fig. 8        ein Diagramm, bei dem die Beschleunigung und somit die zweite Ableitung der die Oberflächenform in Fig. 6 darstellenden mathematischen Funktion in Abhängigkeit vom Azimutwinkel dargestellt ist;

Fig. 9        ein Diagramm, bei dem im Ausführungsführungsbeispiel eine azimutale Funktionsbeschreibung der Oberfläche des Übergangsbereichs dargestellt ist;

Fig. 10        eine perspektivische Darstellung eines weiteren Ausführungsbeispiels einer erfindungsgemäßen Augenlinse, bei welcher der optische Teil mit einem teilweisen Blick auf eine erste und eine zweite optische Oberfläche gezeigt ist und die symmetrische Oberflächenausgestaltung zu erkennen ist;

Fig. 11        eine perspektivische Darstellung einer Oberfläche die den astigmatischen Summandenanteil in der die Gesamtoberfläche beschreibenden Funktion zeigt;

Fig. 12        die Darstellung gemäß Fig. 2 in leicht gedrehter Perspektive zu Fig. 2;

Fig. 13        eine perspektivische Darstellung eines Ausführungsbeispiels einer optischen Oberfläche eines optischen Teils eines Ausführungsbeispiels einer erfindungsgemäßen Augenlinse, die gemäß Fig. 12 ausgebildet ist und zusätzlich die Oberfläche so gestaltet ist, dass die Linse zur Korrektur eines Astigmatismus dient;

Fig. 14 bis 16     Kurvendarstellungen der Oberflächen gemäß Fig. 11 bis 13;

Fig. 17     eine perspektivische Darstellung einer weiteren Oberfläche die den astigmatischen Summandenanteil in der die Gesamtoberfläche beschreibenden Funktion zeigt;

Fig. 18     die Darstellung gemäß Fig. 2 in leicht gedrehter Perspektive zu Fig. 2;

Fig. 19     eine perspektivische Darstellung eines Ausführungsbeispiels einer optischen Oberfläche eines optischen Teils eines Ausführungsbeispiels einer erfindungsgemäßen Augenlinse, die gemäß Fig. 12 ausgebildet ist und zusätzlich die Oberfläche so gestaltet ist, dass die Linse zur Korrektur eines Astigmatismus dient; und

Fig. 20 bis 22     Kurvendarstellungen der Oberflächen gemäß Fig. 17 bis 19.

Bevorzugte Ausführung der Erfindung

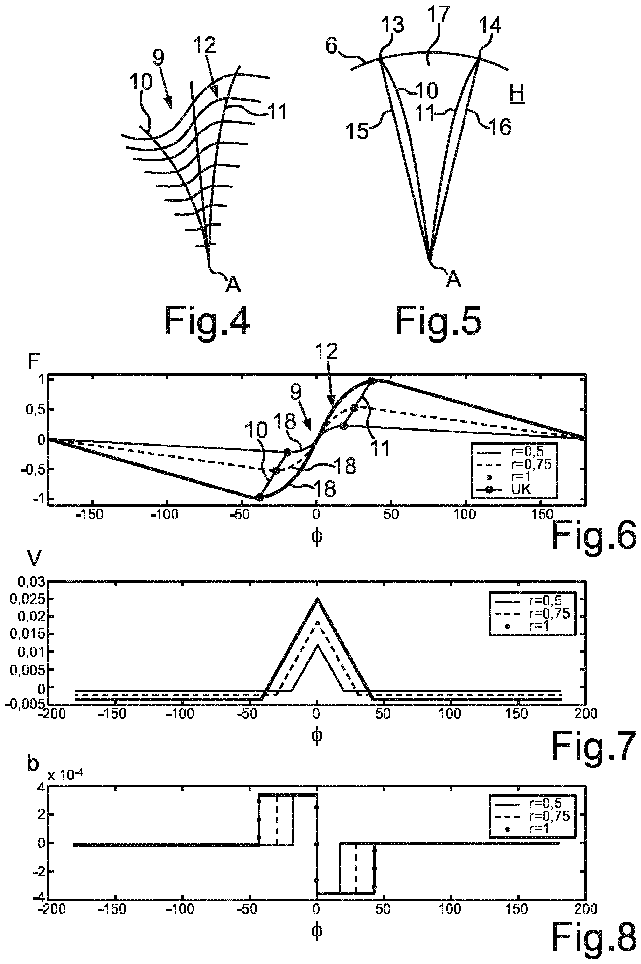

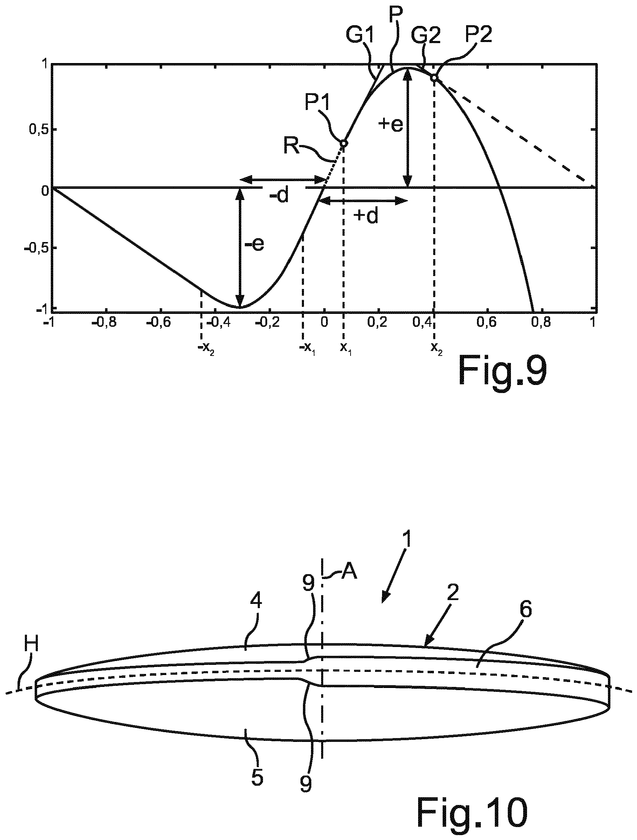

In den Figuren werden gleiche oder funktionsgleiche Elemente mit den gleichen Bezugszeichen versehen.

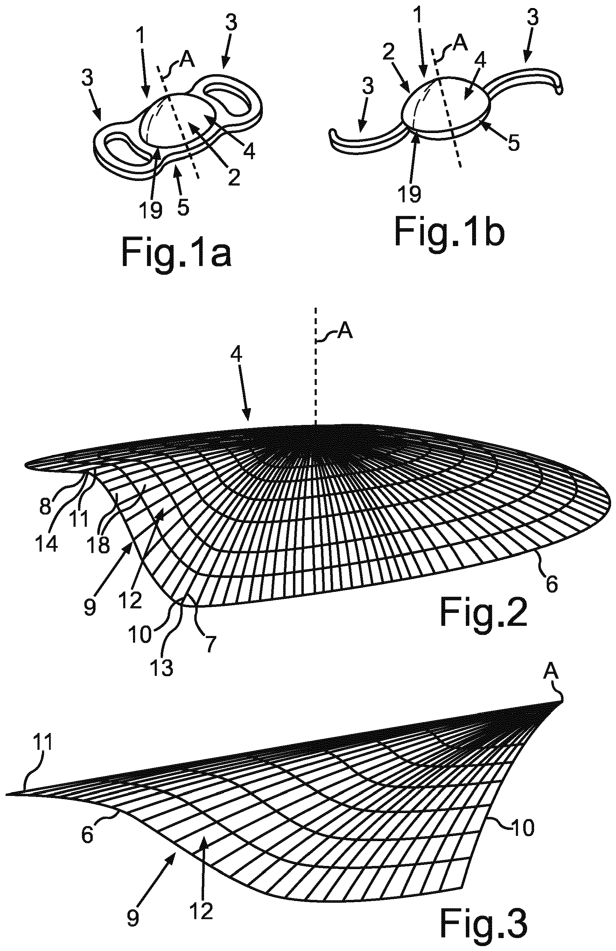

In Fig. 1a ist in einer perspektivischen Darstellung ein erstes Ausführungsbeispiel einer Augenlinse 1 gezeigt, die eine Intraokularlinse ist. Die Augenlinse 1 umfasst einen optischen Teil 2 und daran anschließend eine Haptik 3. Die Augenlinse 1 ist faltbar und über einen Kleinschnitt in ein Auge einführbar.
Der optische Teil 2 umfasst eine optische Hauptachse A, deren Richtung auch die z-Koordinate ist. Der optische Teil 2 weist darüber hinaus eine erste optische Fläche 4 und eine auf der gegenüberliegenden Seite ausgebildete zweite optische Fläche 5 auf.
In Fig. 1b ist in einer perspektivischen Darstellung ein weiteres Ausführungsbeispiel einer als Intraokularlinse ausgebildeten Augenlinse 1 gezeigt. Sie unterscheidet sich von der Ausführung in Fig. 1a durch die unterschiedliche Haptik 3.
In Fig. 2 ist in einer vergrößerten Darstellung ein Ausführungsbeispiel der ersten optischen Oberfläche 4 dargestellt. Sie ist als gekrümmte und um die optische Hauptachse A umlaufende schraubenbahnförmige Windung gestaltet. Es ist eine einzige umlaufende Windung gestaltet, die sich in radialer Richtung und somit senkrecht zur Achse A von der Achse A bis zu einem Umfang 6 ausbildet. Der Umfang 6 stellt somit auch vorzugsweise das äußere Ende des optischen Teils 2 dar.
Die erste optische Oberfläche 4 ist im Hinblick auf ihre Schraubenbahn somit mit einer Ganghöhe ausgebildet, wobei die Windung einen Anfang 7 und ein Ende 8 aufweist.
Darüber hinaus umfasst die erste optische Oberfläche 4 einen Übergangsbereich 9, welcher sich zwischen dem Anfang 7 und dem Ende 8 erstreckt. Der Übergangsbereich 9 ist in radialer Richtung ebenfalls zwischen der Achse A und dem Umfang 6 ausgebildet. In Umlaufrichtung um die Achse A und somit in azimutaler Ausrichtung umfasst der Übergangsbereich 9 einen Anfangsrand 10 und einen Endrand 11. Der Übergangsbereich 9 geht dabei mit seinem Anfangsrand 10 direkt in den Anfang 7 über, wobei auch der Endrand 11 direkt in das Ende 8 übergeht.
Eine Oberfläche 12 des Übergangsbereichs 9 ist stufenlos ausgebildet und auch das Einmünden des Anfangsrands 10 in den Anfang 7 ist stufenlos ausgebildet. Ebenso ist das Einmünden des Endrands 11 in das Ende 8 stufenlos ausgebildet.
Der Anfangsrand 10 erstreckt sich auch hier von der Hauptachse A bis zu einer ersten Umfangsstelle 13. Der Endrand 11 erstreckt sich ebenfalls von der Hauptachse A zu einer zweiten Umfangsstelle 14.
Der Übergangsbereich 9 mit seinem Anfangsrand 10 und seinem Endrand 11 ist so gestaltet, dass bei einer Projektion des Anfangsrands 10 und/oder des Endrands 11 in eine Ebene, insbesondere die Hauptebene H (Fig. 10), senkrecht zur Hauptachse A dieser bzw. diese einen nicht-geradlinigen Verlauf aufweist bzw. aufweisen.
Insbesondere sind der Anfangsrand 10 und/oder der Endrand 11 über ihre gesamte radiale Länge gekrümmt ausgebildet.
Wie in der noch nachfolgend zu erläuternden Projektionsdarstellung in Fig. 5 zu erkennen ist, sind der Anfangsrand 10 und der Endrand 11 ganz spezifisch gestaltet und gekrümmt.
Der Anfangsrand 10 weist einen nicht-geradlinigen Verlauf auf, der gegenüber einer geradlinigen Verbindung zwischen der Hauptachse A und der ersten Umfangsstelle 13 in Umlaufrichtung um die Hauptachse A betrachtet in Richtung des Endrands 11 hin verlaufend ausgebildet ist. Der Anfangsrand 10 liegt in der in Fig. 5 gezeigten Projektionsebene H somit insbesondere über seine gesamte Länge näher zum Endrand 11 als eine geradlinige Verbindung 15 zwischen der Achse A und der ersten Umfangsstelle 13.

Darüber hinaus ist auch der Endrand 11 nicht-geradlinig verlaufend ausgebildet und erstreckt sich zwischen der Achse A und der zweiten Umfangsstelle 14 in der in Fig. 5 gezeigten Projektionsebene H insbesondere über seine gesamte Länge näher zum Anfangsrand 10 als eine geradlinige Verbindung 16 zwischen der Achse A und der zweiten Umfangsstelle 14.

In der in Fig. 5 gezeigten und in der Figurenebene liegenden Projektionsebene H wird somit durch die ganz spezifischen Anordnungen und Krümmungen sowie Gestaltungen des Anfangsrands 10 und/oder des Endrands 11 ein Flächenbereich 17 des Übergangsbereichs 9 in der Projektionsebene H erzeugt, der kleiner ist, als ein Flächenbereich zwischen den geradlinigen Verbindungen 15 und 16.

Gemäß der Darstellung in Fig. 2 ist zu erkennen, dass die Oberfläche 12 des Übergangsbereichs 9 so geformt ist, dass zumindest am Anfangsrand 10, insbesondere über dessen gesamte radiale Länge zwischen der Hauptachse A und der ersten Umfangsstelle 13 ein Verlauf ausgebildet ist, bei dem die in Umlaufrichtung betrachtete Steigung der Oberfläche 12 an allen radialen Stellen des Anfangsrands 10 den gleichen funktionalen und somit mathematisch beschreibbaren Steigungsverlauf aufweisen. Sie haben somit quasi den gleichen Anstieg, der gemäß Fig. 7 linear ist, so dass die Beschleunigung gemäß Fig. 8, welche die Ableitung von Fig. 7 ist, in dem Bereich konstant ist.

Die Ausführung gemäß Fig. 2 bis Fig. 5 ist derart, dass sich der Anfangsrand 10 über die gesamte Länge zwischen der Hauptachse A und der Umfangsstelle 13 erstreckt und somit quasi in diese Endpunkte mündet. Gleiches gilt für den Endrand 11. Es könnte aber auch vorgesehen sein, dass die Oberfläche 4 nicht über den gesamten Bereich mit einer gewundenen Struktur ausgebildet ist, sondern beispielsweise in der Mitte eine Bereich einer nicht gewundenen Struktur ausgebildet ist, beispielsweise eine monofokale Linse, an den radial dann ein weiterer Bereich angrenzt, der eine gewundenen Struktur aufweist. Bei dieser Ausführung erstreckt sich dann der Anfangsrand 10 und der Endrand 11 zwischen der Hauptachse A und den Umfangsstellen, münden jedoch nicht in die Hauptachse sondern enden radial dazu beabstandet, insbesondere an der Grenze zwischen den beiden genannten Bereichen.

Dies ist auch durch die dargestellten Diagramme in Fig. 6 bis 8 gezeigt. Hier sind in Fig. 6 Schnittdarstellungen an unterschiedlichen radialen Stellen am Übergangsbereich 9 gezeigt, wobei das Diagramm in Fig. 6 den Oberflächenverlauf in Abhängigkeit von dem azimutalen Winkel φ darstellt. In den Darstellungen gemäß Fig. 6 bis 8 ist eine Formgebung des Übergangsbereichs dargestellt, die invers zur Darstellung in Fig. 2 und Fig. 3 ausgebildet ist. Dies ist jedoch im Hinblick auf die Realisierung und die dann tatsächlich damit erreichbare Funktionalität unerheblich und beide Ergebnisse und Darstellungen sind im Hinblick auf ihre Wirkung an der Augenlinse 1 identisch.

Insbesondere ist auch vorgesehen, dass die Oberfläche 12 des Übergangsbereichs 9 so geformt ist, dass zumindest am Endrand 11, insbesondere über seine gesamte radiale Länge zwischen der Hauptachse A und der zweiten Umfangsstelle 14 ein Verlauf ausgebildet ist, bei dem die in Umlaufrichtung betrachtete Steigung der Oberfläche 12 an allen radialen Stellen des Endrands 11 den gleichen funktionalen Steigungsverlauf aufweist.

Wie die Darstellungen in Fig. 2 und Fig. 3 sowie in den Fig. 6 bis Fig. 8 verdeutlichen, ist die Oberfläche 12 des Übergangsbereichs 9 an allen radialen Stellen zwischen der Hauptachse A und dem Umfang 6 des optischen Teils 2 mit einem Konturenverlauf 18 in Umlaufrichtung betrachtet ausgebildet, der zumindest bereichsweise nicht-geradlinig ausgebildet ist. Der Konturenverlauf der Oberfläche 12 bezeichnet dabei jene Linie bzw. Kurve, die durch die Oberfläche 12 bei einem Schnitt durch den Übergangsbereich 9 an einer spezifischen radialen Stelle erscheint.

Besonders bevorzugt ist es, dass dieser Konturenverlauf 18, der in Fig. 2 der Übersichtlichkeit dienend nur an einigen Stellen symbolhaft eingezeichnet ist, als Parabelverlauf ausgebildet ist. Ein derartiger Verlauf ist auch in den Diagrammen gemäß Fig. 6 bis Fig. 8 erläutert.

Es ist bei der vorteilhaften Ausführung zu erkennen, dass dieser Konturenverlauf 18 zwischen dem Anfangsrand 10 und dem Endrand 11 symmetrisch zu einem in Umlaufrichtung um die Hauptachse A jeweils im gleichen Winkelabstand zu dem Anfangsrand 10 und dem Endrand 11 gelegenen Symmetriepunkt ist, der in einer Symmetrieebene liegt, die die Hauptachse A umfasst und senkrecht zu der Hauptebene H orientiert ist.

Insbesondere ist der funktionale Steigungsverlauf zwischen dem Anfangsrand 10 und dem Symmetriepunkt an allen radialen Stellen gleich. In einer weiteren vorteilhaften Ausgestaltung ist vorgesehen, dass er über die gesamte Länge zwischen der Achse A und dem Umfang 6 maximal um 10 % variiert.

Vorzugsweise ist vorgesehen, dass z-Werte und somit die Werte der Oberfläche in der Richtung der Hauptachse A der Augenlinse der Oberfläche als Funktion vom Winkel φ, der sich in Umlaufrichtung um die Hauptachse A bemisst und den Azimutwinkel darstellt, allgemein als Summe von Sinus-Funktionen beschreibbar sind, wie dies nachfolgende Formel 1 zeigt:

$$z(r,\varphi) = z_{\max}(r) * z(\varphi) = z_{\max}(r) * \sum a_n \sin(n\varphi) \qquad (1)$$

Bezüglich der weiteren Bestimmung wird auf die Ausführungen zu den Formeln 2 bis 4 vorne verwiesen.

In Fig. 3 ist in einer vergrößerten Darstellung der Übergangsbereich 9 der ersten optischen Oberfläche 4 gemäß Fig. 2 gezeigt.

In Fig. 4 ist darüber hinaus eine perspektivische Draufsicht mit vereinfachter Darstellung des Übergangsbereichs 9 mit den gekrümmt verlaufenden Anfangsrand 10 und Endrand 11 dargestellt.

In den Darstellungen gemäß Fig. 7 und Fig. 8 sind die mathematischen funktionalen Darstellungen der Ableitung sowie der zweiten Ableitung der Oberflächenfunktion, wie sie in Fig. 6 des Übergangsbereichs 9 dargestellt ist, eingezeichnet. Die angesprochenen gleichen funktionalen Steigungsverläufe der symmetrischen Ausgestaltung sind in Fig. 7 gezeigt. Darüber hinaus sind die konstanten zweiten Ableitungen, wie sie an allen radialen Stellen zwischen dem Anfangsrand 10 und der Symmetrieebene einerseits und der Symmetrieebene und dem Endrand 11 andererseits gegeben sind, gezeigt. Aus Fig. 8 ist ersichtlich, dass die Beschleunigung einen Maximalwert a nicht übersteigt, und im gesamten Übergangsbereich 9 zwischen Anfangsrand 10 und Endrand 11 gleich bleibt.

Beispielhaft sind in den Diagrammen gemäß Fig. 6 bis Fig. 8 jeweils Kurvenlinien für die radialen Stellen an dem Umfang 6 (r = 1) und dann für r = 0,75 und r = 0,5 gezeigt.

Die Oberfläche 4 ist darüber hinaus in einer ersten Ausführung so gestaltbar, dass ausgehend von dem Anfang 7 eine Dioptriewertänderung von vorzugsweise drei Dioptrien erreicht wird. Dies wird insbesondere in einem Winkelintervallbereich von 354° ermöglicht, wobei sich darin dieser Dioptriewert kontinuierlich ändert. Vorzugsweise weist der Übergangsbereich 9 eine Winkelbreite von 6° auf, wobei hier diese Winkelbreite am Umfang 6 vorzugsweise bemessen ist.

Insbesondere ist vorgesehen, dass beispielsweise die Brechkraft 21 Dioptrie am Anfang 7 und 24 Dioptrie am Ende 8 beträgt.

Es liegt somit eine Änderung der Brechkraft außerhalb des Umfangsbereichs 9 derart vor, dass sie sich linear als Funktion vom Winkel in Umlaufrichtung um die Hauptachse A ändert.

Im Diagramm in Fig. 9 ist der funktionale Verlauf und somit die mathematisch beschreibende Funktion der Kontur 18 der Oberfläche 12 beispielhaft entsprechend Gleichung (6) gezeigt. Im Unterschied zu einer direkten Aneinanderfügung von zwei Parabeln ist hier zwischen den Parabeln eine geradlinige Ausgestaltung und somit ein entsprechender Konturenverlauf ausgebildet. Es sind jeweils nur die Abschnitte und Stellen im oberen Bereich und somit oberhalb der Horizontalachse gekennzeichnet. Es bezeichnet dabei R den geradlinigen Verlaufsabschnitt der Geraden G1 mit dem Anstieg m1, P1 die Übergangsstelle von der Geraden G1 in die Parabel P, P2 den Übergang von der Parabel in eine Gerade G2 mit dem Anstieg m2. Der Übergangsbereich 9 ist jedoch nur bis zum Punkt P2 ausgebildet, so dass die restliche Parabeldarstellung nur zur mathematischen Beschreibung gezeigt ist. Die Oberfläche folgt ab dem Punkt P2 der Geraden G2. Entsprechendes gilt für den Kurvenverlauf unter der Horizontalachse, der sich aus der Symmetrie um den Punkt $\varphi=0$ ergibt.

In Fig. 10 ist in einer perspektivischen Darstellung ein weiteres Ausführungsbeispiel einer Augenlinse 1 gezeigt. Diese ist im Unterschied zur bisherigen Erläuterung dahingehend gestaltet, dass sowohl die erste optische Oberfläche 4 als auch die zweite optische Oberfläche 5 mit einer einzigen schraubenbahnförmigen Windung ausgestaltet sind und dann ein entsprechender Übergangsbereich 9 an der Ganghöhe gestaltet ist. Darüber hinaus ist vorgesehen, dass die Oberflächen 4 und 5 symmetrisch zur Hauptebene H, die senkrecht zur Achse A steht, gestaltet sind. Bei einer derartigen Ausführung ist somit die Schraubenbahngestaltung der optischen Oberfläche 4, wie sie in Fig. 2 und Fig. 3 bei einer lediglich einseitig entsprechend strukturiert ausgebildeten Augenlinse 1 vorgesehen war, nicht mit einer Dioptriewertänderung von drei auf einer Seite zu gestalten, sondern mit der Hälfte davon. In Addition der beiden symmetrisch ausgebildeten und strukturierten Oberflächen 4 und 5 ergibt sich dann wiederum in Summe eine Dioptriewertänderung von vorzugsweise drei bei einem Umlauf um die Achse A.

Darüber hinaus ist vorgesehen, dass eine Übergangszone 19 (Fig. 1) zwischen dem optischen Teil 2 und der Haptik 3 mit einer Oberfläche ausgebildet ist, deren Kontur in radialer Richtung zumindest bereichsweise eine Kreisbogenform oder eine Parabelform aufweist. Insbesondere ist dieser Übergang bzw. diese Übergangszone 19 zwischen der Kontur des optischen Teils 2 und der Kontur des haptischen Teils 3 ohne einen geradlinigen Konturenverlauf und somit einen entsprechenden Abschnitt ausgebildet.

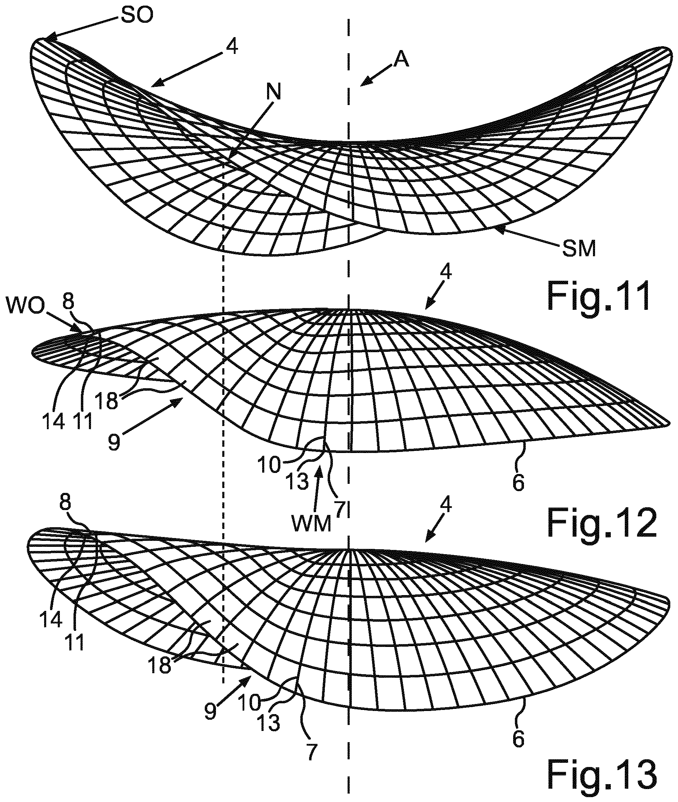

In Fig. 11 ist eine perspektivische Darstellung der Oberfläche gezeigt, die durch den Summanden $a_2 \cdot r^2 \cdot \sin(2 \cdot \varphi)$ für den Astigmatismus erzeugt wird. Die Oberfläche ist als Sattelfläche ausgebildet, so dass sich auf der Linsenoberfläche eine torische Form ergibt.

In Fig. 12 ist eine Darstellung der Oberfläche 4 gemäß Fig. 2 gezeigt, wobei im Vergleich zu Fig. 2 eine dazu leicht gedrehte Ansicht dargestellt ist.

In Fig. 13 ist ein Ausführungsbeispiel einer zur Korrektur eines Astigmatismus ausgebildeten Linse mit dargestellter Oberfläche 4 des optischen Teils gezeigt. Dabei ist die Oberfläche 4 in Fig. 12 in zur Fig. 11 vergleichbaren Position gezeigt, so dass die Unterschiedlichkeit der Oberflächenform zu erkennen ist, zwischen der Linse mit und ohne Korrekturwirkung eines Astigmatismus.

In Fig. 13 ist die Überlagerung der Oberflächen gemäß Fig. 11 und 12 gezeigt. Es ist dabei im Ausführungsbeispiel vorgesehen, dass für die Oberfläche gemäß Fig. 13 ausgehend von der Oberfläche der Fig. 12 eine zusätzliche Sattelfläche gemäß Fig. 11 addiert wird, so dass sich als tatsächliche Gesamtoberfläche die in Fig. 13 bildet.

Insbesondere ist im Ausführungsbeispiel vorgesehen, dass die Sattelfläche, die sich durch den oben genannten Summanden für die Astigmatismuskorrektur ergibt, und die Oberfläche mit der Windung in Umlaufrichtung um die Hauptachse A so zueinander ausgerichtet und zu einer endgültigen an der Linse ausgebildeten Gesamtoberflächenform überlagert sind, dass ein oberer Sattelpunkt SO in Fig. 11 mit der flachsten Krümmung WO in Fig. 12 der Windung zusammenfällt.

Insbesondere ist ein Rotationswinkel in Umlaufrichtung um die Achse A zwischen der Sattelfläche und der Oberfläche mit der Windung so ausgebildet, dass das Maximum (Sattelpunkt SO) der die Sattelfläche beschreibenden Sinusfunktion des Summanden an der Stelle des flacheren Radius (flachste Krümmung WO) der Oberfläche mit der Windung und das Minimum SM der Sinusfunktion in Richtung des steileren Radius WM der Oberfläche mit der Windung liegt bzw. der Nulldurchgang N der Sinusfunktion in der Symmetrieebene des Übergangsbereichs 9 liegt.

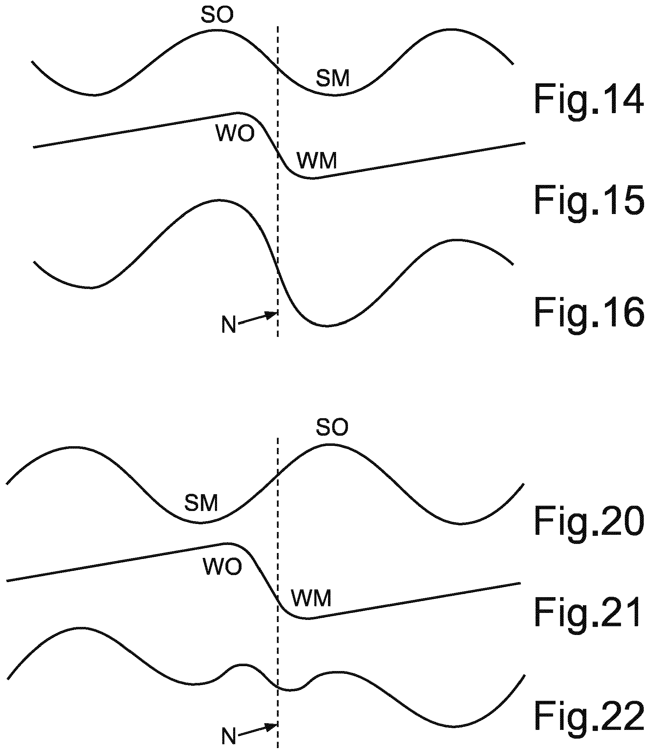

In Fig. 14 bis 16 sind die Kurvenverläufe der Oberflächen gemäß Fig. 11 bis 13 mit der gestrichelten Symmetrieebene dargestellt.

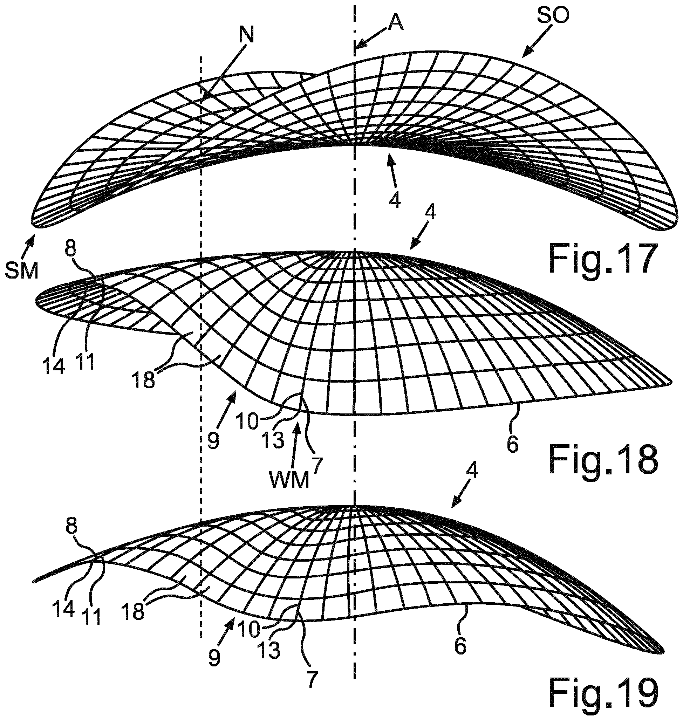

In Fig. 17 ist eine perspektivische Darstellung einer weiteren Oberfläche gezeigt, die durch den Summanden $a_2$*$r^2$*sin (2*$\varphi$) für den Astigmatismus erzeugt wird, jedoch im Vergleich zu Fig. 11 unterschiedlich zur Darstellung in Fig. 11 in Umlaufrichtung um die Achse A positioniert ist. Die Oberfläche ist als Sattelfläche ausgebildet, so dass sich auf der Linsenoberfläche eine torische Form ergibt.

In Fig. 18 ist eine Darstellung der Oberfläche 4 gemäß Fig. 2 und Fig. 12 gezeigt, wobei im Vergleich zu Fig. 2 eine dazu leicht gedrehte Ansicht dargestellt ist.

In Fig. 19 ist ein Ausführungsbeispiel einer zur Korrektur eines Astigmatismus ausgebildeten Linse mit dargestellter Oberfläche 4 des optischen Teils gezeigt. Dabei ist die Oberfläche 4 in Fig. 18 in zur Fig. 17 vergleichbaren Position gezeigt, so dass die Unterschiedlichkeit der Oberflächenform zu erkennen ist, zwischen der Linse mit und ohne Korrekturwirkung eines Astigmatismus.

In Fig. 19 ist die Überlagerung der Oberflächen gemäß Fig. 17 und 18 gezeigt. Es ist dabei im Ausführungsbeispiel vorgesehen, dass für die Oberfläche gemäß Fig. 19 ausgehend von der Oberfläche der Fig. 12 eine zusätzliche Sattelfläche gemäß Fig. 17 addiert wird, so dass sich als tatsächliche Gesamtoberfläche die in Fig. 19 bildet. Im Übrigen gelten die oben dargelegten Erläuterungen.

In Fig. 20 bis 22 sind die Kurvenverläufe der Oberflächen gemäß Fig. 17 bis 19 mit der gestrichelten Symmetrieebene dargestellt.

Die Augenlinse 1 ist insbesondere als Schärfentiefenlinse ausgebildet. Sie ist als refraktive Linse gestaltet. Dies ist insbesondere dahingehend realisiert, dass in radialer Richtung von der Achse A bis zum Umfang 6 kein periodisches Wellen- und Tälerprofil realisiert ist.

**Patentansprüche**

1. Augenlinse (1) mit einem optischen Teil (2), der eine erste optische Oberfläche (4) aufweist, wobei die erste optische Oberfläche (4) zumindest bereichsweise mit einer Struktur mit zumindest einer Windung mit einer Ganghöhe umlaufend um eine Hauptachse (A) der Augenlinse (1) ausgebildet ist, und ein Übergangsbereich (9) zwischen einem Anfang (7) und einem Ende (8) der Windung ausgebildet ist, der mit einem Anfangsrand (10) und einem Endrand (11) in die Windung übergeht, wobei sich der Anfangsrand (10) zwischen der Hauptachse (A) und einer ersten Umfangsstelle (13) und der Endrand (11) zwischen der Hauptachse (A) und einer zweiten Umfangstelle (14) erstreckt, **dadurch gekennzeichnet, dass** der in eine Ebene (H) senkrecht zur Hauptachse (A) projizierte Anfangsrand (10) einen nicht-geradlinigen Verlauf aufweist und/oder der in eine Ebene (H) senkrecht zur Hauptachse (A) projizierte Endrand (11) einen nicht-geradlinigen Verlauf aufweist.

2. Augenlinse (1) nach Anspruch 1, **dadurch gekennzeichnet, dass** der Anfangsrand (10) und/oder der Endrand (11) über ihre gesamte Länge gekrümmt ist bzw. sind.

3. Augenlinse (1) nach Anspruch 1 oder 2, **dadurch gekennzeichnet, dass** der Anfangsrand (10) einen nicht-geradlinigen Verlauf aufweist, der gegenüber einer geradlinigen Verbindung (15) zwischen der Hauptachse (A) und der ersten Umfangsstelle (13) in Umlaufrichtung um die Hauptachse (A) betrachtet in Richtung des Endrands (11) hin verlaufend ausgebildet ist und/oder der Endrand (11) einen nicht-geradlinigen Verlauf aufweist, der gegenüber einer geradlinigen Verbindung (16) zwischen der Hauptachse (A) und der zweiten Umfangsstelle (14) in Umlaufrichtung um die Hauptachse (A) betrachtet in Richtung des Anfangsrands (10) hin verlaufend ausgebildet ist.

4. Augenlinse (1) nach einem der vorhergehenden Ansprüche, **dadurch gekennzeichnet, dass** eine Oberfläche (12) des Übergangsbereichs (9) so geformt ist, dass zumindest am Anfangsrand (10), insbesondere über seine gesamte Länge, zwischen der Hauptachse (A) und der ersten Umfangstelle (13) ein Verlauf ausgebildet ist, bei dem die in Umlaufrichtung betrachtete Steigung der Oberfläche (12) an allen radialen Stellen des Anfangsrands (10) den gleichen Steigungsverlauf aufweist.

5. Augenlinse (1) nach einem der vorhergehenden Ansprüche, **dadurch gekennzeichnet, dass** eine Oberfläche (12) des Übergangsbereichs (9) so geformt ist, dass zumindest am Endrand (11), insbesondere über seine gesamte Länge, zwischen der Hauptachse (A) und der zweiten Umfangstelle (14) ein Verlauf ausgebildet ist, bei dem die in Umlaufrichtung betrachtete Steigung der Oberfläche (12) an allen radialen Stellen des Endrands (11) den gleiche Steigungsverlauf aufweist.

6. Augenlinse (1) nach einem der vorhergehenden Ansprüche, **dadurch gekennzeichnet, dass** eine Oberfläche (12) des Übergangsbereichs (9) einen Konturenverlauf (18) in einem Schnitt im radialen Abstand zur Achse (A) und durch den Übergangsbereich (9) betrachtet aufweist, der zumindest bereichsweise nicht-geradlinig ausgebildet ist.

7. Augenlinse (1) nach Anspruch 6, **dadurch gekennzeichnet, dass** der Konturenverlauf (18) zumindest bereichsweise eine Parabel ist oder der Konturenverlauf (13) zumindest bereichsweise durch eine kubische Funktion beschrieben ist oder zumindest bereichsweise einen Kreisbahnabschnitt darstellt.

8. Augenlinse (1) nach Anspruch 6 oder 7, **dadurch gekennzeichnet, dass** der Konturenverlauf (18) zwischen dem Anfangsrand (10) und dem Endrand (11) symmetrisch zu einer in Umlaufrichtung um die Hauptachse (A) jeweils in gleichem Winkelabstand zu dem Anfangsrand (10) und dem Endrand (11) gelegenen Symmetrieebene ist.

9. Augenlinse (1) nach Anspruch 4 oder 5 und Anspruch 8, **dadurch gekennzeichnet, dass** der Steigungsverlauf zwischen dem Anfangsrand (10) und der Symmetrieebene und/oder der Steigungsverlauf zwischen dem Endrand (11) und der Symmetrieebene an allen radialen Stellen gleich ist.

10. Augenlinse (1) nach einem der vorhergehenden Ansprüche, **dadurch gekennzeichnet, dass** ein in die senkrecht zur Hauptachse (A) stehende Ebene (H) projizierter und durch den projizierten Anfangsrand (10) und den projizierten Endrand (11) begrenzter Flächenbereich (17) des Übergangsbereichs (9) kleiner ist, als eine Fläche in dieser Ebene (H) die durch zwei geradlinige Flächenränder (15, 16) begrenzt ist, von denen sich einer von der Hauptachse (A) durch die erste Umfangsstelle (13) und der weitere von der Hauptachse (A) durch die zweite Umfangsstelle (14) erstreckt.

11. Augenlinse (1) nach einem der vorhergehenden Ansprüche, **dadurch gekennzeichnet, dass** die Asphärizität der ersten optischen Oberfläche (4) um die Hauptachse (A) variiert, insbesondere kontinuierlich sich vergrößert.

12. Augenlinse (1) nach einem der vorhergehenden Ansprüche, **dadurch gekennzeichnet, dass** zumindest eine Oberfläche (4, 5) des optischen Teils (2) so gestaltet ist, dass sich die Brechkraft des optischen Teils (2) in Umlaufrichtung um die Hauptachse (A) bei einer Umdrehung zwischen 1 Dioptrie und 5 Dioptrie, insbesondere zwischen 1 Dioptrie und 4 Dioptrie ändert, insbesondere kontinuierlich ändert und/odersich die Brechkraft des optischen Teils (2) außerhalb des Übergangsbereichs (9) linear als Funktion vom Winkel in Umlaufrichtung um die Hauptachse (A) ändert.

13. Augenlinse (1) nach einem der vorhergehenden Ansprüche, **dadurch gekennzeichnet, dass** der Übergangsbereich (9), insbesondere zwischen den beiden Umfangsstellen (13, 14) gemessen, in Umlaufrichtung um die Achse (A) eine Winkelbreite kleiner 7°, insbesondere zwischen 3° und 6,5°, aufweist.

14. Augenlinse (1) nach einem der vorhergehenden Ansprüche, **dadurch gekennzeichnet, dass** die erste optische Oberfläche (4) so gestaltet ist, dass als Sehfehler ein Astigmatismus korrigierbar ist und/oder dass sie eine Tiefenschärfenlinse ist.

15. Augenlinse (1) nach einem der vorhergehenden Ansprüche, **dadurch gekennzeichnet, dass** eine rückwärtige zweite optische Oberfläche (5) des optischen Teils (2) symmetrisch zur ersten optischen Oberfläche (4) in Bezug zur senkrecht zur Hauptachse (A) stehenden Ebene (H) ausgebildet ist.

**Claims**

1. Ocular lens (1) with an optical part (2) that has a first optical surface (4), wherein the first optical surface (4) is embodied, at least in regions, with a structure with at least one winding with a pitch that is circumferential about a principal axis (A) of the ocular lens (1), and a transition region (9) is embodied between a start (7) and an end (8) of the winding, said transition region merging into the winding with a start edge (10) and an end edge (11), the start edge (10) extending between the principal axis (A) and a first circumferential spot (13) and the end edge (11) extending between the principal axis (A) and a second circumferential spot (14), **characterized in that** the start edge (10) projected into a plane (H) perpendicular to the principal axis (A) has a non-linear profile and/or the end edge (11) projected into a plane (H) perpendicular to the principal axis (A) has a non-linear profile.

2. Ocular lens (1) according to Claim 1, **characterized in that** the start edge (10) and/or the end edge (11) is or are curved over their entire length.

3. Ocular lens (1) according to Claim 1 or 2, **characterized in that** the start edge (10) has a non-linear profile, which is embodied extending towards the direction of the end edge (11) in relation to a linear connection (15) between the principal axis (A) and the first circumferential spot (13) when seen in the circumferential direction about the principal axis (A), and/or the end edge (11) has a non-linear profile, which is embodied extending towards the direction of the start edge (10) in relation to a linear connection (16) between the principal axis (A) and the second circumferential spot (14) when seen in the circumferential direction about the principal axis (A).

4. Ocular lens (1) according to any one of the preceding claims, **characterized in that** a surface (12) of the transition region (9) is shaped in such a way that a profile in which the gradient of the surface (12) seen in the circumferential direction has the same gradient profile at all radial spots of the start edge (10) is formed at least on the start edge (10), in particular over its entire length, between the principal axis (A) and the first circumferential spot (13).

5. Ocular lens (1) according to any one of the preceding claims, **characterized in that** a surface (12) of the transition region (9) is shaped in such a way that a profile in which the gradient of the surface (12) seen in the circumferential direction has the same gradient profile at all radial spots of the end edge (11) is formed at least on the end edge (11), in particular over its entire length, between the principal axis (A) and the second circumferential spot (14).

6. Ocular lens (1) according to any one of the preceding claims, **characterized in that** a surface (12) of the transition region (9) has a contour profile (18) that has, at least in regions, a non-linear embodiment when seen in a section at a radial distance from the axis (A) and through the transition region (9).

7. Ocular lens (1) according to Claim 6, **characterized in that** the contour profile (18), at least in regions, is a parabola or the contour profile (13), at least in regions, is described by a cubic function or, at least in regions, represents a circular path portion.

8. Ocular lens (1) according to Claim 6 or 7, **characterized in that** the contour profile (18) between the start edge (10) and the end edge (11) is symmetrical in relation to a plane of symmetry lying in a circumferential direction about the principal axis (A), in each case at the same angular distance from the start edge (10) and the end edge (11).

9. Ocular lens (1) according to Claim 4 or 5 and Claim 8, **characterized in that** the gradient profile between the start edge (10) and the plane of symmetry and/or the gradient profile between the end edge (11) and the plane of symmetry is the same at all radial spots.

10. Ocular lens (1) according to any one of the preceding claims, **characterized in that** a surface area (17) of the transition region (9) that is projected into the plane (H) perpendicular to the principal axis (A) and that is delimited by the projected start edge (10) and the projected end edge (11) is smaller than a surface in this plane (H) that is delimited by two linear surface edges (15, 16), one of which extends from the principal axis (A) through the first circumferential spot (13) and the further one of which extends from the principal axis (A) through the second circumferential spot (14).

11. Ocular lens (1) according to any one of the preceding claims, **characterized in that** the asphericity of the first optical surface (4) varies about the principal axis (A), in particular increases continuously.

12. Ocular lens (1) according to any one of the preceding claims, **characterized in that** at least one surface (4, 5) of the optical part (2) is designed in such a way that the refractive power of the optical part (2) changes, in particular changes continuously, between 1 dioptre and 5 dioptres, in particular between 1 dioptre and 4 dioptres, in the circumferential direction about the principal axis (A) during one revolution and/or the refractive power of the optical part (2) outside of the transition region (9) changes linearly as a function of the angle in the circumferential direction about the principal axis (A).

13. Ocular lens (1) according to any one of the preceding claims, **characterized in that** the transition region (9) has an angular width of less than 7°, in particular of between 3° and 6.5°, in the circumferential direction about the axis (A), in particular as measured between the two circumferential spots (13, 14).

14. Ocular lens (1) according to any one of the preceding claims, **characterized in that** the first optical surface (4) is designed in such a way that an astigmatism as a refractive error is correctable and/or that it is a depth-of-field lens.

15. Ocular lens (1) according to any one of the preceding claims, **characterized in that** a back second optical surface (5) of the optical part (2) has a symmetric embodiment with respect to the first optical surface (4) in relation to the plane (H) that is perpendicular to the principal axis (A).

**Revendications**

1. Lentille ophtalmique (1), pourvue d'une partie optique (2), qui comporte une première surface optique (4), la première surface optique (4) étant conçue au moins par zones avec une structure dotée d'au moins une convolution avec un pas périphérique autour d'un axe principal (A) de la lentille ophtalmique (1), et au moins une zone de transition (9) entre un début (7) et une fin (8) de la convolution qui passe par un bord de début (10) et par un bord de fin (11) dans la convolution, le bord de début (10) s'étendant entre l'axe principal (A) et un premier emplacement circonférentiel (13) et le bord de fin (11) s'étendant entre l'axe principal (A) et un deuxième emplacement circonférentiel (14), **caractérisée en ce que** le bord de début (10) projeté dans un plan (H) à la perpendiculaire de l'axe principal (A) présente un trajet non rectiligne et/ou le bord de fin (11) projeté dans un plan (H) à la perpendiculaire de l'axe principal (A) présente un trajet non rectiligne.

2. Lentille ophtalmique (1) selon la revendication 1, **caractérisée en ce que** le bord de début (10) et/ou le bord de fin (11) est ou sont incurvé (s) sur toute leur longueur.

3. Lentille ophtalmique (1) selon la revendication 1 ou 2, **caractérisée en ce que** le bord de début (10) présente un trajet non rectiligne, qui par rapport à un assemblage rectiligne (15) entre l'axe principal (A) et le premier emplacement circonférentiel (13), considéré dans la direction circonférentielle autour de l'axe principal (A) est conçu en s'écoulant en direction du bord de fin (11) et/ou **en ce que** le bord de fin (11) présente un trajet non rectiligne, qui par rapport à un assemblage rectiligne (16) entre l'axe principal (A) et le deuxième emplacement circonférentiel (14), considéré dans la direction circonférentielle autour de l'axe principal (A) est conçu en s'écoulant en direction du bord de début (10).

4. Lentille ophtalmique (1) selon l'une quelconque des revendications précédentes, **caractérisée en ce qu'**une surface (12) de la zone de transition (9) est façonnée de telle sorte qu'au moins sur le bord de début (10), notamment sur toute sa longueur, entre l'axe principal (A) et le premier emplacement circonférentiel (13) soit conçu un trajet sur lequel l'inclinaison de la surface (12) considérée dans la direction circonférentielle présente sur tous les emplacements radiaux du bord de début (10) le même trajet d'inclinaison.

5. Lentille ophtalmique (1) selon l'une quelconque des revendications précédentes, **caractérisée en ce qu'**une surface (12) de la zone de transition (9) est façonnée de telle sorte qu'au moins sur le bord de fin (11), notamment sur toute sa longueur, entre l'axe principal (A) et le deuxième emplacement circonférentiel (14) soit conçu un trajet sur lequel l'inclinaison de la surface (12) considérée dans la direction circonférentielle présente sur tous les emplacements radiaux du bord de fin (11) le même trajet d'inclinaison.

6. Lentille ophtalmique (1) selon l'une quelconque des revendications précédentes, **caractérisée en ce qu'**une surface (12) de la zone de transition (9) présente une forme de contour (18) dans une coupe à un écart radial par rapport à l'axe (A) et considérée à travers la zone de transition (9) qui est conçue au moins par emplacements de manière non rectiligne.

7. Lentille ophtalmique (1) selon la revendication 6, **caractérisée en ce que** la forme de contour (18) est au moins par endroits une parabole ou **en ce que** la forme de contour (13) est décrite au moins par endroits par une fonction cubique ou représente au moins par endroits un segment de trajectoire circulaire.

8. Lentille ophtalmique (1) selon la revendication 6 ou 7, **caractérisée en ce que** la forme de contour (18) entre le bord de début (10) et le bord de fin (11) est symétrique à un plan de symétrie situé chaque fois sous un écart angulaire identique par rapport au bord de début (10) et au bord de fin (11) dans la direction circonférentielle autour de l'axe principal (A).

9. Lentille ophtalmique (1) selon la revendication 4 ou 5 et la revendication 8, **caractérisée en ce que** le trajet d'inclinaison entre le bord de début (10) et le plan de symétrie et/ou le trajet d'inclinaison entre le bord de fin (11) et le plan de symétrie est identique sur tous les emplacements radiaux.

10. Lentille ophtalmique (1) selon l'une quelconque des revendications précédentes, **caractérisée en ce qu'**une zone de surface (17) de la zone de transition (9), projetée dans le plan (H) debout à la perpendiculaire de l'axe principal (A) et délimitée par le bord de début (10) projeté et par le bord de fin (11) projeté est inférieure à une surface dans ledit plan (H) qui est délimitée par deux bords de surface (15, 16) rectilignes, dont l'un s'étend à partir de l'axe principal (A) à travers le premier emplacement circonférentiel (13) et l'autre s'étend à partir de l'axe principal (A) à travers le deuxième emplacement circonférentiel (14).

11. Lentille ophtalmique (1) selon l'une quelconque des revendications précédentes, **caractérisée en ce que** l'asphéricité de la première surface optique (4) autour de l'axe principal (A) varie, notamment augmente en continu.

12. Lentille ophtalmique (1) selon l'une quelconque des revendications précédentes, **caractérisée en ce qu'**au moins une surface (4, 5) de la partie optique (2) est conçue de telle sorte que lors d'une rotation, le pouvoir de réfraction de la partie optique (2) dans la direction circonférentielle autour de l'axe principal (A) varie entre 1 dioptrie et 5 dioptrie, notamment entre 1 dioptrie et 4 dioptrie, notamment varie en continu et/ou **en ce que** le pouvoir de réfraction de la partie optique (2) hors de la zone de transition (9) varie de manière linéaire, en tant que fonction de l'angle dans la direction circonférentielle autour de l'axe principal (A).

13. Lentille ophtalmique (1) selon l'une quelconque des revendications précédentes, **caractérisée en ce que** la zone de transition (9) présente, mesurée notamment entre les deux emplacements circonférentiels (13, 14), dans la direction circonférentielle autour de l'axe (A) une largeur angulaire inférieure à 7°, notamment comprise entre 3° et 6,5°.

14. Lentille ophtalmique (1) selon l'une quelconque des revendications précédentes, **caractérisée en ce que** la première surface optique (4) est conçue de telle sorte que le défaut visuel corrigible soit un astigmatisme et/ou de sorte à être une lentille à profondeur de champ.

15. Lentille ophtalmique (1) selon l'une quelconque des revendications précédentes, **caractérisée en ce qu'**une deuxième surface optique (5) arrière de la partie optique (2) est conçue de manière symétrique à la première surface optique (4), en rapport au plan (H) debout à la perpendiculaire de l'axe principal (A).

Fig.1a

Fig.1b

Fig.2

Fig.3

Fig.4

Fig.5

Fig.6

Fig.7

Fig.8

Fig.9

Fig.10

SO

4

A

N

SM

**Fig.11**

WO   8

4

14  11   18

9

10

13

WM   7

6

**Fig.12**

8

4

14

11

18

9   10

13

7

6

**Fig.13**

Fig.17

Fig.18

Fig.19

Fig.14

Fig.15

Fig.16

Fig.20

Fig.21

Fig.22

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013045079 A1